# EUROPEAN PATENT SPECIFICATION

(11) **EP 3 232 819 B1**
(45) Date of publication and mention of the grant of the patent: **22.05.2019**
(21) Application number: 15813827.1
(22) Date of filing: 17.12.2015
(51) Int. Cl.: A23L 33/00

(54) **INFANT NUTRITION WITH HYDROLYSED PROTEIN AND PALMITIC ACID**
BABYNAHRUNG MIT HYDROLYSIERTEM PROTEIN UND PALMITINSÄURE
NUTRITION DE NOURRISSON AVEC UNE PROTÉINE HYDROLYSÉE ET DE L'ACIDE PALMITIQUE

(30) Priority: 19.12.2014 US 201462094190 P
(43) Date of publication of application: 25.10.2017
(73) Proprietor: Nestec S.A., 1800 Vevey (CH)
(72) Inventor: KLINE, Randi, Malvern, Pennsylvania 19355 (US); O'REGAN, Jonathan, Cork City (IE)
(74) Representative: Gagliardi, Tatiana
(86) International application number: PCT/EP2015/080359
(87) International publication number: WO 2016/097221

(56) References cited:
- EP-A1- 2 143 340
- WO-A1-2006/114791
- WO-A1-2007/088160
- WO-A1-2009/016632
- WO-A1-2011/135564
- DE-A1-102007 022 694

## Description

### Technical field of the invention

The present invention relates to a composition for use in formulaes for infants or young children comprising partially hydrolysed protein and an oil mix comprising palmitic acid wherein the palmitic acid esterified in the sn-2 position of triacylglycerols is in the amount of from 20% to 60% by weight of total palmitic acid and palmitic acid esterified in the sn-1(3) position of triacylglycerols is in the amount of from 40% to 80% by weight of total palmitic acid. Furthermore, the invention relates to an infant formula comprising said composition and the use of said composition or infant formula.

### Background of the invention

Mother's milk is recommended for all infants as a sole source of nutrition for up to 4-6 months of age, since human breast milk supplies vital nutrients to infants. However, in some cases breastfeeding is inadequate or unsuccessful or inadvisable for medical reasons, or the mother chooses not to breast feed either at all or for a period of more than a few weeks. Infant formulations have been developed for these situations.

Research into the components of human milk has been going on for many years and it is by no means complete even now. The research however shows that there are differences between the nutrients present in human milk and the ones used in the commercially available infant formulas on the market today. For example, the type of lipid composition can differ significantly. The lipids present in human breast milk contain for example a large amount of palmitic acid esterified on the sn-2 (beta) position of the triacylglycerol, in the range of 68-72%. In infant formulas, vegetable oils are typically used as the lipid source to match the high palmitic acid content in human milk. The source of palmitic acid in the infant formulas used today comprises a high amount (i.e. 80%) of palmitic acid esterified in the sn-1(3) (alpha) position of the triacylglycerol and thus a low amount of palmitic acid esterified in the sn-2 position.

This difference in oils and fats and in particular the difference in the regiospecific distribution of palmitic acid causes a difference in the digestion of fats in infants. Infants fed with infant formulations comprising an oil mix comprising palmitic acid having a high amount of said palmitic acid in the sn-1(3) position of the triacylglycerols have been shown to lead to an increased calcium excretion in the faeces as compared to infants fed with a human breast milk. Palmitic acid in the sn-1 and sn-3 positions are more likely to be cleaved and form free fatty acids. Free palmitic acid can bind calcium and form insoluble calcium-palmitic acid complexes (referred to as palmitic acid soaps) in the intestine. Excretion of these soaps in the feces may be partially responsible for harder stools experienced by some formula fed infants. Harder stools are a component of functional constipation in infants and young children.

Further, calcium loss in a population such as preterm infants, may increase the risk of insufficient bone mineralization.

Some studies are known where infants fed with infant formulas comprising a high proportion of sn-2 palmitate, such as more than 66% sn-2 palmitate of total amount of palmitic acid (and lower than 34% sn-1(3) palmitate) were observed to have a significant reduction of calcium excreted in the faeces as compared to infants fed with a standard formula. On the contrary, infants fed with an infant formula having a more moderate content of sn-2 palmitate, approximately 44% of total palmitic acid, showed no significant decrease in faecal calcium as compared to infants fed with a standard formula.

In Carnielli et al "Feeding premature newborn infants with palmitic acid in amounts and stereoisomeric position similar to that of human milk; effects on fat and mineral balance", Am J Clin Nutr. May 1995 vol. 61 no. 5, page 1037-1042 is disclosed that an infant formula comprising sn-2 palmitate in an amount closer to that in human breast milk resulted in a lower calcium excretion as compared to when infants were fed with an infant formula having a high amount of sn-1(3) palmitate.

In Carnielli et al "Structural position and amount of palmitic acid in infant formulas: effects on fat, fatty acid, and mineral balance" J. Pediatr Gastroenterol Nitr. 1996, Dec;23(5):553-60, is disclosed a study showing that infants fed with an infant formula comprising 24% palmitic acid having 66% esterified in the sn-2 position of triacylglycerols, would have an faecal calcium excretion significantly lower than for infants fed with an infant formula comprising intermediate and low amounts of sn-2 palmitate out of total palmitic acid (24% palmitic and 39% esterified in the sn-2 position of triacylglycerols) and (20% palmitic acid having 13% esterified in the sn-2 position of triacylglycerols).

Furthermore, studies have shown that infant formulations comprising an amount of 36% of total palmitic acid esterified in the sn-2 position of triacylglycerols and lower and thus 64% of palmitic acid esterified in the sn-1(3) position of triacylglycerols and higher, will result in no significant decrease in the faecal calcium excretion.

Thus, the palmitic acid as well as its regiospecific distribution (sn-2 versus sn-1(3)) is important for reduction of calcium excreted in faeces.

International Patent application WO2007/088160 relates to a nutritional composition for VLBW (very low birth weight) infants which comprises 26 to 38g/L of a source of hypoallergenic hydrolysed whey protein with a degree of hydrolysis between 8 and 20, 37 to 46 g/l of a fat source of which 20 to 50% is medium chain triglycerides and which has an n3:n6 ratio between 6 and 12 and 50 to 100g/l of a carbohydrate source which composition contains between 3.2 and 4.0 grams of proteins per 100Kcal. Such document indicates that the fat soruce may comprise from 5 to 20% of a structured lipid in which at least 50% of the palmitic residues contained in the structured lipid are esterified at the sn2 position.

German patent application DE102007022694 relates to a food supplement for use in improving calcium uptake with contains a combination of milk fat with milk protein and /or hydrolysed milk protein. Such document indicates that milk fat contains esterified palmitic acid of which most preferably 40-45%are esterified at the sn2 position.

However, preparations containing palmitic acid with a high amount esterified in the sn-2 position of triacylglycerols are complex and expensive to prepare. Therefore, due to manufacturing limitations, current commercially available formulas for infants and young children comprising palmitic acid esterified to triacylglycerols comprises a moderate amount of palmitic acid esterified in sn-2 position of the triacylglycerols. These formulas do not show a benefit in reduced calcium excreted.

Therefore, there is a need in the art for a composition for use in formulations to infants or young children comprising a moderate (20-60%) amount of palmitic acid esterified in the sn-2 position of the triacylglycerol, but which composition when consumed results in faecal calcium excretion which is close to the calcium excretion obtained when consuming either human milk or formulas having a high (above 65%) proportion of palmitic acid esterified in the sn-2 position of the triacylglycerols.

### Summary of the invention

Thus, an object of the present invention is to provide a composition for use in formulas for infants and young children, and in human milk fortifier, which composition even though having a moderate amount of sn-2 palmitate results in low amounts of calcium excreted in the stool, and which amounts of excreted calcium is close to the amounts of calcium excreted by infants being fed with human breast milk.

In particular, it is an object to provide a composition with a moderate content of sn-2 palmitate which can have a high calcium absorption and low palmitic acid soap formation.

In particular, it is an object of the present invention to provide a composition that solves the above mentioned problems of the prior art.

Thus, one aspect of the invention relates to a composition as described in claim 1.

Another aspect of the present invention is to provide an infant formula comprising the composition according to the invention and wherein the ratio between the partially hydrolysed protein and the sn-2 palmitate is from 1.5:1 to 35:1 based on weight.

Yet another aspect of the present invention relates to the composition or infant formula according to the invention for use in administration to an infant or young child to reduce calcium excretion by said infant or young child.

Still another aspect of the present invention relates to the composition or infant formula according to the invention for use in administration to an infant or young child to improve calcium homestasis, increase calcium absorption, increase calcium retention, increase calcium utilization and/or reduce the formation of palmitic acid soaps.

In still another aspect, the present invention relates to the composition or infant formula according to the invention for use in administration to an infant or young child to soften stools, prevent and/or reduce the risk of hard stools, prevent and/or reduce the risk of constipation, improve feeding tolerance, decrease the frequency and duration of crying and fussing, and alleviate digestive discomfort and colic.

In still another aspect, the present invention relates to the composition or infant formula according to the invention for use in administration to an infant or young child to improve sleep duration, improve sleep quality and quantity, improve infant and parental quality of life and reduce maternal anxiety.

In a further aspect, the present invention relates to the composition or infant formula according to the invention for use in administration to an infant or young child to increase bone mineralization, increase bone strength, and/or increase bone mineral density.

The present invention will now be described in more detail in the following.

### Detailed description of the invention

### Definitions

Prior to discussing the present invention in further details, the following terms and conventions will first be defined:
Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9 and so forth. All references to singular characteristics or limitations of the present invention shall include the corresponding plural characteristic or limitation, and vice versa, unless otherwise specified or clearly implied to the contrary by the context in which the reference is made.

In the context of the present invention, the term "ratio" by weight (weight/weight) refers to the ratio between the weights of the mentioned compounds. For example, a mixture comprising 60 g whey and 40 g casein would have a weight ratio which is equal to 60:40, which is equal to 3:2 or 1.5 (that is 3 divided with 2). Similarly, a mixture of 50 g whey and 50 g casein would have a ratio by weight of whey and casein of 50:50, which is equal to 1:1 or 1 (that is 1 divided with 1).

The term "and/or" used in the context of the "X and/or Y" should be interpreted as "X", or "Y", or "X or Y".

As used in this specification, the words "comprising", "comprises" and similar words are not to be interpreted in an exclusive or exhaustive sense. In other words they are intended to mean "including, but not limited to".

Unless defined otherwise, all technical and scientific terms used herein have the same meaning as commonly understood by one of ordinary skill in the art.

The term "infant" will in the context of the present invention mean a child under the age of 12 months.

The term "young child" refers to a child in the age from 12 months to 4 years.

In the context of the present invention, the infant may be any term infant or preterm infant. In an embodiment of the invention, the infant is selected from the group of preterm infants and term infants.

The term "infant formula" as used in the context of the present invention refers to a nutritional composition intended for infants during the first months of life and as defined in *Codex Alimentarius,* (Codex STAN 72-1981) and Infant Specialities (incl. Food for Special Medical purposes) as defined in *Codex Alimentarius,* (Codex STAN 72-1981).

The term "follow-on formula" are given to formulas designed to be used from the age of 6 to 12 months of age. The follow-on formula is typically given in combination with the infant is given other types of food, such as vegetable and/or fruit pure.

The term "growing-up milk" is given to formulas designed to be used from the age of one year onwards. It is generally a milk-based beverage adapted for the specific nutritional needs of young children.

The term "composition for use in formulaes for infants or young children" refers in the context of the present invention to either a formula as such, i.e an infant formula, which comprises all nutrients necessary in order to meet the standards of being an infant formula as defined in the Codex Alimentarius. Further, the "composition for use in formulaes for infants or young children" may be a composition comprising nutrients, which together with other compositions of nutrients can be mixed to prepare a formula, i.e. such "composition for use in infant formulation" can be added to a mixture, which is intended to be used as an infant formula. Further, the "composition for use in formulaes" can be a supplement or a fortifier to an infant formula, follow-on formula, growing-up milk, or human milk.

### Oil mix:

In the context of the present invention, the term "oil mix" refers to a mixture of vegetable fats and oils, dairy fats and/or animal fats comprising triacylglycerols as the main source of lipids. The oil mix may be a mixture of one or more vegetable oils and fats, for example selected from the group consisting of palm oil, palm kernel oil, esterified vegetable oils, such as interesterified palm oil, animal fat and animal fat fractions. The oil mix may also comprise other vegetable oils and fats suitable for infant nutrition. For example the oil mix may comprise one or more selected from the group of soyabean oil, coconut oil, rapeseed oil, canola oil, sunflower oil, safflower oil and milk fat.

In an aspect of the present invention, the oil mix used comprises palmitic acid, wherein the relative proportion of palmitic acid esterified in the sn-2 position of triacylglycerol range from 20% to 60% by weight of total palmitic acid and palmitic acid esterified in the sn-1(3) position of triacylglycerol range from 40% to 80% by weight of total palmitic acid. Thus, the proportion of palmitic acid in sn-2 position of the triacylglyceol is at least 20% by weight based on total palmitic acid and the proportion of palmitic acid in sn-2 position of the triacylglyceol is no more than 60% by weight.

### Palmitic acid:

In the context of the present invention, the term "palmitic acid" refers to the saturated fatty acid, 16:0. The term "palmitate" may also be used for "palmitic acid" when esterified to glycerol such as in triacylglycerols. Most palmitic acid in lipid compositions are esterified to triacylglycerols and may be positioned on either the external (sn-1(3)) or internal (sn-2) position of the triacylglycerols. When palmitic acid is esterified in the (external) first or third position of a triacylglycerol, the palmitic acid is in the sn-1(3) position of the triacylglycerol. Such palmitic acid is in the context of the present invention referred to as sn-1(3) palmitate. Palmitic acid esterified in the internal position of a triacylglycerol is a palmitic acid with the position of palmitic acid in the sn-2 position of the triacylglycerol, and such palmitic acid is in the context of the present invention referred to as sn-2 palmitate. The oil mix according to the present invention contains triacylglycerols and some of the triacylglycerols has palmitic acid esterified.

In the context of the present invention, the term "amount sn-2 palmitic acid" refers to the amount of palmitic acid being esterified in the sn-2 position of triacylglycerol based on the total amount of palmitic acid.

Similarly, the term "amount sn-1(3) palmitic acid" refers to the amount of palmitic acid being esterified in the sn-1(3) position of triacylglycerol based on the total amount of palmitic acid.

In an embodiment according to the present invention, the oil mix comprises palmitic acid in an amount of at least 8% by weight based on the total amount of fatty acids, such as at least 10% by weight, preferably at least 12% by weight, such as at least 15% by weight, even more preferably at least 18% by weight.

In a further embodiment according to the present invention, the oil mix comprises palmitic acid in an amount of from 8% to 30% by weight, such as in an amount of from 10 to 26% palmitic acid by weight, preferably in an amount of from 12 to 25% by weight. In particular, the oil mix comprises from 20 to 24% by weight of palmitic acid.

In human milk, more than 98% of fat is in form of triacylglycerols which contain saturated and unsaturated fatty acids. The main saturated fatty acid in human milk is palmitic acid which account for 22-25% of the total amount of fatty acids.

In human milk, about 68-72% of the palmitic acid is esterifed in sn-2 position of triacylglycerol.

In palm olein, which is a palm oil fraction widely used in infant formulation comprising a high amount of palmitic acid, the amount of sn-1(3) palmitate is 90-95% and thus only about 5-10% of palmitic acid is esterified in the sn-2 position of triacylglycerols.

In most commercially available infant formulas, the major part, about 80-90% of the palmitic acid present, is esterified in the sn-1(3) position of triacylglycerols, i.e. is present as sn-1(3) palmitate.

However, it has been found out that the position of palmitic acid in triacylglycerols has an influence on the absorption of minerals, such as for example absorption of calcium, because free palmitic acid forms complexes with minerals. When palmitic acid is esterified in the sn-1(3) position of triacylglycerols, free palmitic acid during digestion is released from the triacylglycerol and forms insoluble complexes with minerals such as calcium if sufficiently present in the diet (this is called palmitic acid soaps). These palimitic acid soaps are not absorbable and are therefore lost in the faeces. The loss of palmitic acid soaps leads to mineral loss as well as poor nutrient absorption.

Palmitic acid soaps may be expressed in many ways, for example as calcium soaps, palmitate soaps, calcium-fatty acid complexes or insoluble complexes. These terms may be used interchangeable, but should be understood as the same.

On the contrary, when palmitic acid is esterified in sn-2 position of the triacylglycerols, unsaturated fatty acids esterified in sn-1(3) position of the triacyglycerols are released during digestion and well-absorbed, therefore avoiding palmitic acid soap formation. Furthermore, sn-2 palmitate is well-absorbed as monoacylglycerol. Hence, if a large amount of palmitic acid is in sn-1(3) position of triacylglycerol, a large amount of calcium will be excreted in the faeces. Studies has shown that using an oil mix comprising palmitic acid having an amount of sn-2 palmitate in a high amount, such as in an amount similar to the content in human milk, preferably above 65-70% will result in an increased calcium absorption as compared to using oil mixes having a more moderate content of sn-2 palmitate.

A high amount of sn-2 palmitate is considered to be an amount of sn-2 palmitate close to the amount present in human milk, i.e. a high amount of sn-2 palmitate is considered to be in the range of 68-80% by weight of total palmitic acid, such as at least above 65% sn-2 palmitate of total palmitic acid.

The inventors of the present invention have surprisingly found out that hydrolysed proteins have a synergistic effect on excretion of calcium when combined with sn-2 palmitate lipids. The inventors have found out that when an oil mix comprising sn-2 palmitates in a moderate amounts, such as about 32-44% by weight of total palmitate, are digested in combination with partially hydrolysed protein, the calcium excretion is close to the calcium excretion seen when feeding infants with a formulation comprising an oil mix having a high (70-80%) amount of sn-2 palmitate, but without the presence of hydrolysed proteins.

As mentioned above conventional vegetable oils rich in palmitic acid, such as for example palm oil fractions, comprises most of the palmitic acid in sn-1(3) position on the triacyglycerols. For example, palm olein comprises 90-95% of palmitic acid esterified in the sn-1(3) position of the triacylglycerols and only about 5-10% in the sn-2 position of the triacylglycerols. Preparing a vegetable oil having a high amount of sn-2 palmitate therefore needs a modification such as for example an enzymatic interesterification process which is complex and expensive.

However, the inventors of the present invention has surprisingly found out that an oil mix with a lower amount of sn-2 palmitate (20-60%) than the amount of sn-2 palmitate present in human breast milk (68-70% in human milk), could result in a calcium excretion close to the one for infants fed with human milk, if partially hydrolysed protein was added to the composition comprising an oil mix with the lower amount of sn-2 palmitate.

Thus, one aspect of the present invention is to provide a composition for use in infant formulation comprising partially hydrolysed protein and an oil mix comprising palmitic acid, wherein the palmitic acid comprises from 20% and up to 60% by weight of sn-2 palmitate and from 40% and up to 80% by weight of sn-1(3) palmitate.

In an embodiment according to the invention, the oil mix comprises sn-2 palmitate in an amount of 2-15% of total fatty acids present in the oil mix and sn-1(3) palmitate in an amount of 5-14% of total fatty acids in the oil mix.

In a further embodiment of the invention, the oil mix comprises palmitic acid, wherein the amount of palmitic acid comprises from 25% to 55% by weight of sn-2 palmitate and from 45% to 75% by weight of sn-1(3) palmitate. In a preferred embodiment, the palmitic acid comprises sn-2 palmitate in an amount of 30% to 50% by weight and sn-1(3) palmitate in an amount of 50% to 70% by weight, such as sn-2 palmitate in an amount of 32% to 44% by
weight and sn-1(3) palmitate in an amount of 56% to 68% by weight.

An oil mix comprising the specific amount of sn-2 palmitate may be prepared by mixing different vegetable oils or by mixing vegetable oils with milk fat. An example of an oil mix could be a mix of cow's milk fat and one or more of vegetable oils, such as sn-2 palmitate, palm oil, coconut oil, soybean oil, high oleic safflower oil and sunflower oil. These fats and oils may be blended in ratios sufficient to provide a similar fatty acid profile to that found in human milk.

A commercially available composition sold by Lipid Nutrition is Betapol™ B-55, which is a triglyceride mixture derived from vegetable oil in which at least 54% of the palmitic acid is in the sn-2 position of the glycerol molecule. In one embodiment, the oil mix present in the compositon according to the present invention is a mix of Betapol™ B-55 and other vegetable oils. Those skilled in the art will know that the percentage of the sn-2 palmitic acid used and the total amount of palmitate in the formula may vary, and that a different sn-2 palmitate oil may be used, without departing form the spirit and scope of the invention.

In another embodiment of the invention, the oil mix furthermore comprises stearic acid, i.e. triacylglycerols having stearic acid attached thereto through ester bonds.

### Protein:

In an aspect of the invention, the composition comprises partially hydrolysed protein.

The inventors of the present invention have surprisingly found out that hydrolysed whey proteins have a positive influence on the calcium excretion and that hydrolysed whey proteins in combination with palmitic acids can decrease the palmitic acid soap formation even though the amount of sn-2 palmitic acids is moderate. It has surprisingly been found that infants fed with a formula containing hydrolysed protein and triacylglycerols esterified with palmitic acid and wherein the palmitic acid comprises less than 50% sn-2 palmitate shows a similar calcium excretion as the infants fed with human milk.

Without being bound by any theory, the inventors of the present invention believes that peptides resulting from hydrolysing a protein may have a less protective effect against proteolysis of digestive enzymes as compared to intact protein. Thus, peptides from protein hydrolysates may not protect the digestive enzyme lipase from proteolysis during digestion while intact proteins will protect lipase. Thus, hydrolysed protein peptides in combination with an oil mix comprising palmitic acid will result in less release of sn-1(3) palmitic acid by lipase, and thus prevent soap formation and improve calcium absorption.

In the context of the present invention, the term "protein" refers to one or more proteins.

The protein used in the present invention may be any protein suitable for being administered to infants. The source of protein may for example be one or more selected from the group of whey protein, casein, soybean protein, pea protein, rice protein or protein from legumes. However, it is preferred that the protein source is based on cow's milkproteins such as whey, casein or mixtures thereof.

In an embodiment of the present invention, the protein is a dairy protein and in a preferred embodiment, the protein is a combination of casein and whey protein.

Casein is a dairy protein commonly found in mammalian milk. Casein makes up about 80% of the proteins in cow's milk and about 40% of the proteins in human milk.

Whey protein is a mixture of globular proteins isolated from whey, which is a liquid material made as a by-product under cheese making. Whey proteins makes up about 20% of the proteins in cow's milk, where about 60% of the proteins in human milk is whey proteins.

Whey proteins is typically a mixture of many different proteins, for example betalactoglobulin, alpha-lactalbumin, lactoferrin and bovine serum albumin and immunoglobulins.

In another preferred embodiment of the invention, the protein is whey protein.

The whey protein may be in the form of for example a whey protein isolate (WPI) or a whey protein concentrate (WPC).

In an aspect of the present invention, the protein is partially hydrolysed protein.

In an embodiment of the present invention, the partially hydrolysed protein is partially hydrolysed whey protein.

The term "hydrolysed protein" refers to protein that has been subjected to hydrolysis to break down the protein into a mixture of peptides and free amino acids. Hydrolysis of proteins is commonly know and can be done by many different methods, for example by enzymatic hydrolysis by using an enzyme such as pancreatic protease to stimulate the naturally occurring hydrolytic process. Hydrolysis may also be by prolonged boiling in a strong acid or a strong base. In the context of the present invention, the partially hydrolysed protein may be produced by any suitable method known in the art. For example, a whey protein hydrolysate may be prepared by enzymatically hydrolysed the whey fraction in two steps as described in EP 0322589A1. For extensively hydrolysing protein, the whey protein may be subjected to triple hydrolysis using first the enzyme Alcalase 2.4 L (EC 940459), then Neutrase 0.5L and then pancreatic enzyme at the temperature about 55°C.

An example of a whey protein hydrolysate which could be used in the present invention is a whey protein hydrolysate comprising about 79% protein and has a degree of hydrolysation being about 13%.

In the context of the present invention "partially hydrolysed protein" refers to proteins, which has been subjected to hydrolysis, where only a portion of the peptide bonds in the protein has been hydrolysed. The partially hydrolysis of protein is measured as the degree of hydrolysis.

The term "degree of hydrolysis" of a protein refers to the number of peptide bonds in the intact protein which are cleaved during the hydrolysis divided by the number of peptide bonds in the intact protein expressed as a percentage.

The term "intact" means in the context of the present invention, proteins where the molecular structure of the protein(s) is not altered according to the conventional meaning of intact proteins. By the term "intact" is meant that the main part of the proteins are intact, i.e. the molecular structure is not altered, for example at least 95% of the proteins are not altered, such as at least 98% of the proteins are not altered.

In an embodiment of the invention the partially hydrolysed protein has a degree of hydrolysation from 8 to 24%, preferably from 11 to 18%, such as from 13 to 16%.

Partially hydrolysed protein comprises protein where essentially all protein has been hydrolysed to some degree. However, the partially hydrolysed protein may comprise some intact protein, where the peptide bonds in some proteins under hydrolysis is broken. In the context of the present invention, a partially hydrolysed protein hydrolysate comprises at most 10% intact protein, such as at most 5% intact protein, preferably at most 3% intact protein, even more preferably at most 1% intact protein. The term "essentially all" refers to that the main part has been hydrolysed, such as at least 95% of the protein has been hydrolysed preferably at least 98% of the protein has been hydrolysed.

In an embodiment of the invention, the composition, for example an infant formula, may comprise both a partially hydrolysed protein hydrolysate and another protein source which comprises intact protein. For example, the source of protein may comprise both intact protein and partially hydrolysed proteins, such as a for example a whey protein isolate. Further, the composition or infant formula according to the present invention may comprise a partially hydrolysed protein hydrolysate and another protein source, said other protein source comprising intact protein. For example partially hydrolysed whey protein isolate and skim milk protein.

The amount of hydrolysed protein should be at least 50% based on total protein, such as at least 60%, preferably at least 70%, such as at least 80%, even more preferably at least 90%.

Skim milk (may also be called skimmed milk) is a product made from whole milk where essentially all milk fats are removed from the product. In skim milk the amount of fat is from zero to 0.3%. Thus, skim milk will comprise both whey protein and casein.

Another example can be a combination if partially hydrolysed whey protein isolate and whole milk protein.

In an embodiment of the invention, the partially hydrolysed protein is present in an amount of at least 5g per litre, such as least 7g per litre, preferably at least 9g per litre. For example, the protein is present in the composition in an amount of 5g to 35g per litre, such as from 8g to 30g per litre, preferably 9 to 17 g per litre or 15 to 30 g per litre.

In a further embodiment of the present invention, the ratio between the partially hydrolysed protein and sn-2 palmitate is from 1.5:1 to 35:1 by weight, such as from 2.0:1 to 30:1, preferably from 2.5:1 to 25:1, even more preferably from 2.5:1 to 15:1, such as from 2.5:1 to 10:1 by weight.

In a further embodiment of the present invention, the ratio between the partially hydrolysed protein and sn-1(3) palmitate is from 2:1 to 20:1 by weight, such as from 2.5:1 to 15:1, preferably from 3:1 to 10:1 by weight.

### Calcium:

In an embodiment of the present invention, the composition furthermore comprises ionic calcium.

The ionic calcium may be any calcium source suitable to be administrated to infants. For example, the ionic calcium may be one or more selected from the group consisting of calcium citrate, calcium hydroxide, calcium oxide, calcium chloride, calcium carbonate, calcium gluconate, calcium phosphate, calcium diphosphate, calcium triphosphate, calcium glycerophosphate, calcium lactate, calcium sulphate.

In a preferred embodiment, the ionic calcium is one or more selected from the group of calcium hydroxide, calcium chloride and calcium citrate.

However, the ionic calcium may also be one or more from the group of calcium phosphate, calcium gluconate and calcium carbonate.

In the context of the present invention, ionic calcium refers to calcium which is not attached to proteins. Ionic calcium may also be known as free calcium. Ionic calcium can be measured with a ion meter.

Calcium phosphate as such is not ionic, but a chemical reaction is made under digestion which releases some free calcium. For example calcium diphosphate follows the following solubilituy equilibrium:

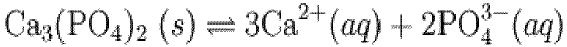

Thus, in the context of the present invention calcium phosphate is considered as a source of ionic calcium.

In an embodiment of the invention, the composition comprises ionic calcium in an amount of at least 1.7 mmol/L.

The inventors of the present invention has surprisingly found out that ionic calcium content in combination with a hydrolysed protein hydrolysate and an oil mix with sn-2 palmitate provides an improved effect on calcium excretion and thus may improve calcium absorption. Without being bound by any theory, the inventors of the present invention believes that ionic calcium provides a significant calcium-peptide interaction with peptides from hydrolysed protein and thus an indirect impact on the different digestive enzymes. The calcium-peptides will cause a poor protection of lipase as compared to intact proteins and peptides not bound to calcium. Lipase is responsible for the release of sn-1(3) palmitic acids from triacylglycerols under digestion, and less sn-1(3) palmitic acids will be released when having a blend of hydrolysed protein and calcium. This results in less formation of palmitic acid soaps and thus more calcium which can be absorbed.

Further ionic calcium and magnesium are able to form bridges between the negatively charged molecules of proteins and increase drastically the formation of heavy precipitates of whey proteins. These calcium and magnesium bridges form much more easily during the heat denaturation which favours a more flexible open structure of the protein chains.

In a further embodiment of the invention, the composition comprises ionic calcium in an amount of at least 2.0 mmol/l , such as for example at least 2.2 mmol/L, preferably at least 2.5 mmol/L.

In a further embodiment of the present invention, the composition comprises ionic calcium in an amount of 1.7 to 5.0 mmol/L, such as 2.0 to 4.5 mmol/L, preferably 2.5 to 4.2 mmol/L, such as 3.0 to 4.0 mmol/L.

### Infant formula:

In an aspect, the composition according to the invention is an infant formula.

However, in another aspect, the composition according to the invention is a fortifier or supplement to human breast milk or to an infant formula or the composition is a composition to be used in making up an infant formula.

In the context of the present invention, the term "infant formula" refers to an infant formula comprising the nutrients normally required for infants to obtain a suitable growth and wherein said infant formula comprises proteins, carbohydrates, lipids, vitamins, minerals and trace elements.

The infant formula according to the present invention may be a starter formula for infants from the age of birth to 4 to 6 months and which provide complete nutrition for this age group (both for term and preterm infants). Further, the infant formula may be a follow-on formula for infants between the ages of four to six months and twelve months which are fed to the infants in combination with increasing amounts of the foods, such as infant cereals and pureed fruits, vegetables and other foodstuffs as the process of weaning progresses.

The infant formula according to the present invention comprises a oil mix, wherein said oil mix comprises palmitic acid and wherein the relative proportion of sn-2 palmitate is 20% to 60% based on total palmitic acid, and the amount of sn-1(3) palmitate is 40% to 80% of total palmitic acid.

In an embodiment, the sn-2 palmitate is 2-15% of total fatty acids in the oil mix and the sn-1(3) palmitate is 5-14% of total fatty acids in the oil mix.

The oil mix may comprise other fatty acids than palmitic acid, mainly these fatty acids are bound to triacylglycerols, but some may be present as free fatty acids. Examples of other fatty acids present is monounsaturated or saturated fatty acids, such as oleic acid or stearic acid and polyunsaturated fatty acids, such as linoleic acid, alpha-linolenic acid, myric acid, arachidonic acid, and docosahexaenoic acid.

In a preferred embodiment, the oil mix comprises stearic acid.

Typically, the infant formula comprises an oil mix in an amount of 20 to 45 g per litre infant formula.

All the features and embodiments relating to the oil mix, palmitic acid, hydrolysed protein and ionic calcium described above in connection with the composition according to the invention also applies to the infant formula.

Thus, in an embodiment, the infant formula comprises an oil mix, wherein the oil mix comprises palmitic acid in an amount of 15 to 25% by weight, such as in an amount of 18 to 22% by weight.

The protein content in the infant formula (term and preterm) is typically between 1.6 to 3.6 g/100 kcal, but may be lower. The protein content in the starter formulaes are in the lower range and the protein content of the follow-on formulas being in the upper end of the range.

In a further embodiment, the infant formula comprises proteins in an amount of 5.0 to 35.0 g per litre.

As mentioned earlier, the protein present in the infant formula may not all be hydrolysed protein, but a part of the protein in the infant formula may be another protein source which for example is intact. However, there must be a certain amount of hydrolysed protein present in the infant formula to obtain the desired effect. Thus, the amount of partially hydrolysed proteins in the infant formula is at least 50% of the total amount of protein, such as at least 60% of the total amount of protein, preferably at least 70% of the total amount of protein, even more preferably at least 80% of the total protein. In an embodiment, the protein present in the infant formula is essentially all partially hydrolysed protein.

By the term "essentially all" is meant that a major part, i.e. essentially all protein is partially hydrolysed, for example at least 95% is partially hydrolysed, such as at least 98%.

The infant formula according to the present invention may also comprise a source of carbohydrates. The composition may comprise one or more carbohydrate. The preferred source of carbohydrate is lactose although other carbohydrate such as saccharose, maltodextrin, and starch may also be added. Preferably, the carbohydrate present in the infant formula according to the present invention is between 9 and 14 g/ 100 kcal. The carbohydrate present in the infant formula is preferably lactose.

The infant formula may also comprise all vitamins and minerals understood to be essential in the daily diet in nutritionally significant amounts. Minimum requirements have been established for certain vitamins and minerals. Examples of minerals, vitamins and other nutrients present in the nutritional composition include vitamin A, vitamin B1, vitamin B2, vitamin B6, vitamin E, vitamin K, vitamin C, vitamin D, folic acid, inositol, niacin, biotin, pantothenic acid, choline, calcium, phosphor, iodine, iron, magnesium, copper, zinc, manganese, chloride, potassium, sodium, selenium, chromium, molybdenum, taurine, and L-carnitine. The minerals are usually added in the salt form.

Other components may be added to an infant formula, such as probiotics, prebiotics, emulsifiers and stabilizers.

### Preparation:

The composition and infant formula according to the present invention may be prepared by any known or otherwise suitable manner. For example, an infant formula may be proposed by blending together a source of protein with a carbohydrate source and a lipid source in appropriate proportions. If used, emulsifiers may be included at this stage. Vitamins and minerals may be added at this stage, but may also be added later to avoid thermal degradation. Water, preferably water which has been subjected to reverse osmosis or deionized water, may then be added and mixed in to form a liquid mixture. The temperature of mixing is preferably room temperature, but may also be higher. The liquid mixture may then be thermally treated to reduce bacterial loads. The mixture may then be homogenized.

If it is desired to produce a powdered composition, the homogenized mixture is dried in a suitable drying apparatus, such as a spray drier or freeze drier and converted into powder.

Processes used in the manufacture of formulae for infants and young children are based on the concept that the products must be nutritionally adequate and microbiologically safe to consume. Thus, steps that eliminate or restrict microbiological growth are central to production processes. The processing technology for each specific formula is proprietary to the manufacturer but, in general, it involves the preservation of an oil-in-water (o/w) emulsion by dehydration in the case of powder products or, sterilization in the case of ready-to-feed or concentrated liquid products. Powdered infant formula may be produced using various processes, such as dry blending dehydrated ingredients to constitute a uniform formula or hydrating and wet-mixing a mixture of macro-ingredients, such as fat, protein and carbohydrate ingredients and then evaporating and spray drying the resultant mixture. A combination of the two processes described above may be used where a base powder is first produced by wet-mixing and spray drying all or some of the macro-ingredients and then dry blending the remaining ingredients, including carbohydrate, minerals and vitamins and other micronutrients, to create a final formula. Liquid formulae are available in a ready-to-feed format or as a concentrated liquid, which requires dilution, normally 1:1, with water. The manufacturing processes used for these products are similar to those used in the manufacture of recombined milk.

If it is desired to produce a liquid infant formula, the homogenized mixture is filled into suitable containers, preferably aseptically. However, the liquid composition may also be retorted in the container, suitable apparatus for carrying out the filling and retorting of this nature is commercially available.

### Use of the composition/infant formula:

The present invention is also directed to the composition or infant formula according to the present invention for use in administration to an infant or young child to reduce calcium excretion by said infant or young child. The composition or infant formulaes are administered to the infant by feeding the infant.

The infant could be any infant, i.e. both a term and preterm infant. The composition or infant formula according to the invention may advantageously be administered to infants or young children. In a particular embodiment, it is used for infants of less than 12 months, i.e. less than 6 months or less than 3 months. In one embodiment, the composition or infant formula is a preterm infant formula. In another embodiment, the composition or infant formula is designed for consumption by infants from birth to 12 months.

Further, the present invention is directed to the composition or infant formula according to the present invention for use in administration to an infant or young child to improve calcium homeostasis, increase calcium absorption, increase calcium retention, increase calcium utilization and/or reduce the formation of palmitic acid soaps.

Without being bound by theory, the increased calcium absorption is thought to have a beneficial physiological effect as well as an effect on bone mineralisation. The inventors of the present invention has surprisingly found out that when using hydrolysed proteins in combination with an oil mix comprising moderate amounts palmitate in sn-2 position and high amounts in sn1 position, palmitic acid and calcium are less excreted in stools than when using a formula containing an oil mix with 40-44% sn-2 palmitic acid and intact protein. The hydrolysed proteins are without being bound by any theory believed to influence the effect of pancreatic lipase which releases palmitic acid from the sn-1(3) position of triacylglycerols, such that less sn-1(3) palmitate is released from the triacylglycerol. Thus, when hydrolysed proteins are present, it will result in less sn-1(3) palmitate released and thus less free palmitic acids available to form soaps with calcium. Reduced palmitic acid soaps may lead to reduced constipation and improved gastrointestinal tolerance as compared to a standard infant formula. Hence, when less calcium is excreted, more calcium may be absorbed.

Similar effects on calcium excretion can be obtained with a formula containing hydrolysed protein and an oil mix with a moderate amount of palmitic acid in sn-2 position of triacylglycerol (20-60%) as obtained with human milk.

The present invention is also directed to a composition or infant formulation according to the invention for use in administration to an infant or young child to soften stools, prevent and/or reduce the risk of hard stools, prevent and/or reduce the risk of constipation, decrease the frequency and duration of crying and fussing and alleviate digestive discomfort, and colic. A reduction in digestive discomfort can improve infant sleep duration, improve sleep quality and quantity, improve improve infant and parental quality of life and reduce maternal anxiety.

Increased calcium absorption and retention can lead to increased bone mineralization. Increased calcium absorption is due to less formation of palmitic acid soaps which are poorly absorbed, but on the contrary excreted. These palmitic acid soaps (calcium-fatty acid complexes) when excreted in the stool may cause the stool to be hard stool. Thus, an object of the present invention is also softening of stools.

Furthermore, an aspect of the present invention relates to the composition or infant formulation according to the present invention for use in administration to an infant or young child to increase bone mineralization, bone strength, and bone mineral density.

Especially, for preterm infants or low or very low birth weight infants have a high incidence of metabolic bone disease, 30-50%, and there are therefore at need to increase bone mineralization in this type of infants. Metabolic bone disease increases the risk of fractures and growth failure. Inadequate calcium absorption is believed to be a main factor in metabolic bone disease.

It should be noted that embodiments and features described in the context of one of the aspects of the present invention also apply to the other aspects of the invention.

The invention will now be described in further details in the following non-limiting examples.

### Examples

The following examples illustrates the specific embodiments of the composition and infant formula according to the invention and the use of said composition and infant formula. The examples are given solely for the purpose of illustration and are not to be construed as limitations of the present invention, as many variations thereof are possible.

### Example 1

Example 1 describes a comparison of different studies conducted to study infants being fed with different infant formulas compared to a control infant formula In group I, infants are fed with an infant formula comprising intact proteins and an oil mix comprising triacylglycerols with palmitic acid, with 35.9% of palmitic acid in the sn-2 position of triacylglycerol. The infants were fed with said infant formula for four weeks and then palmitate soaps (calcium-palmitate complexes), total fatty acid soaps and calcium in the stool were measured and compared to amounts found in the stool of infants fed with a control infant formula.

In group II, infants, as in group I, are fed with an infant formula comprising intact proteins and an oil mix comprising triacylglycerols with 35.9% of palmitic acid in sn-2 position of triacylglycerol. The infants were fed with said infant formula for eight weeks and then palmitate soaps (calcium-palmitate complexes), total fatty acid and calcium in the stool were measured and compared to the amounts in a control infant formula.

In group III, infants are fed with an infant formula comprising partially hydrolysed protein and an oil mix comprising triacylglycerols with 35.9% of palmitic acid in sn-2 position of triacylglycerol. The infants were fed with said infant formula for eight weeks and then palmitate soaps (calcium-palmitate complexes), total fatty acid and calcium in the stool were measured and compared to the amounts in a control infant formula.

The control infant formula comprises an oil mix having sn-2 palmitate in an amount of about 11.7% of total palmitic acid. Further, the control formula comprises a standard intact cow's milk protein as the protein source.

The calcium content in stool is also measured for a group of infants fed with human milk.

The primary objective of the studies was to measure the amount of calcium and fatty acid in the stool of infants fed with the different infant formulas as compared to a control infant formula.

Both the three test formulas (in group I, II, and III) and the control formula was prepared as a ready-to-feed liquid. The three test formulas and the control formula are described below in table 1.

**Table 1:**

| | Test formula in group I | Test formula in Group II | Test formula in group III | Control formula |
|---|---|---|---|---|
| Amount palmitic acid in oil mix | 8.2 g/L 22.8% | 8.2 g/L 22.8% | 8.2 g/L 22.8% | 7.5 g/L 21.3% |
| Amount sn-2 palmitate of total palmitic acid | 35.9% | 35.9% | 35.9% | |
| Amount sn-2 palmitate (% of total fat) | 8.2% | 8.2% | 8.2% | 2.6 |
| Amount Sn-1(3) palmitate (% of total fat) | 14.9% | 14.9% | 14.9% | 18.8 |

The calcium excreted in the stools were measured for the three groups and in the control formula.

Table 2 below shows the calcium content in stool measured in the faeces of infants in group I, II and III. Further, table 2 shows the calcium stool content in infants fed with the control and in infants fed with human breast milk. Table 2 furthermore shows the calcium content in the different infant formulas.

**Table 2:**

| | Group I infant formula vs. control | | | Group II infant formula vs. control | | | Group III infant formula vs. control | | |
|---|---|---|---|---|---|---|---|---|---|
| Stool Calcium (% change) | ↑ 3.7% | | | ↓ 5.2% | | | ↓ 20.5% | | |
| | NS | | | NS | | | (p<0.05) | | |
| Stool calcium (mean mg/g | Test 39.4 | Ctrl 38 | HM 17.6 | Test 30.7 | Ctrl 32.4 | HM 20.3 | Test 25.6 | Ctrl 32.3 | ND |
| Calcium content in test formula (mg/100 ml) | Test 42 | Ctrl 42 | HM NA | Test 46 | Ctrl 47 | HM NA | Test 46 | Ctrl 47 | ND |

| | | | | | | | | | |
|---|---|---|---|---|---|---|---|---|---|
| NS= not significant; ND=not done; NA=not applicable | | | | | | | | | |

From table 2 it can be seen that the calcium content in the test formulas and control formulas are almost similar, i.e. no significant difference.

Furthermore, table 2 shows that the infants fed the test formula in group III have a faecal calcium content more similar to infants fed with human breast milk than infants fed test formula in group I and II. Thus, infants fed with an infant formula comprising moderate amounts of sn-2 palmitate and partially hydrolysed proteins has a content of calcium excreted in stools more similar to infants fed with human breast milk as compared to infants fed with infant formulas with a high amount of sn-2 palmitate and intact proteins.

Besides from the calcium content excreted in the stools, the fatty acid soaps were also measured for the three groups. The content of calcium in stools and the fatty acid soaps were measured and compared to the contents in the control formula. The data can be seen in table 3.

**Table 3:**

| | Group I infant formula vs. control | Group II infant formula vs. control | Group III infant formula vs. control |
|---|---|---|---|
| Palmitic acid soaps | ↓15% | ↓36% | ↓41 % |
| | (p=0.0028) | (p<0.0001) | (p<0.0001) |
| Total fatty acid soaps | ↓ 8% | ↓40% | ↓ 28% |
| | (NS) | (p=0.0001) | (p<0.01) |
| Calcium in stool | ↑ 3.7% | ↓ 5.2% | ↓ 20.5% |
| | (NS) | (NS) | (p<0.05) |

| | | | |
|---|---|---|---|
| ↓: refers to a reduction as compared to the control. ↑: refers to an increase as compared to the control. | | | |

The infants in the test groups are fed with the test infant formulas as the sole nutrition.

From table 3, it is shown that the infant formula comprising a combination of sn-2 palmitate (35.9%) and hydrolysed protein has an improved effect on reducing the amount of palmitic acid soaps as compared to infants fed with an infant formula comprising sn-2 palmitate in an amount of 35.9% and intact proteins. Thus, it is shown that hydrolysed proteins have an effect on calcium excretion in stools when administered with sn-2 palmitate in moderate amounts. The calcium excretion for the infants in group III was reduced by 20.5% as compared to infants fed with the control infant formula, while there was no significant difference in the calcium excretion for the infants in group I and II. Further, the faecal calcium content for the infants fed in group III was closer to the faecal calcium content in infants fed with human breast milk.

## Claims

1. A composition for use in formulaes for infants or young children comprising partially hydrolysed protein and an oil mix comprising palmitic acid esterified to triacylglycerols, wherein the palmitic acid esterified in the sn-2 position of triacylglycerol is in the amount of from 20% to 60% by weight of total palmititic acid and palmitic acid esterified in the sn-1(3) position of triacylglycerol is in the amount of from 40% to 80% by weight of total palmitic acid; and wherein the ratio between the partially hydrolysed protein and the sn-2 palmitate is from 1.5:1 to 35:1 based on weight

2. Composition according to claim 1, wherein the partially hydrolysed protein is partially hydrolysed whey protein.

3. Composition according to claim 1, wherein the partially hydrolysed protein has a degree of hydrolysation from 8% to 24%.

4. Composition according to any of the claims 1 to 3, wherein the composition furthermore comprises ionic calcium.

5. Composition according to claim 4, wherein the ionic calcium is one or more selected from the group consisting of calcium citrate, calcium hydroxide, calcium oxide, calcium chloride, calcium carbonate, calcium gluconate, calcium phosphate, calcium diphosphate, calcium triphosphate, calcium glycerophosphate, calcium lactate, and calcium sulphate.

6. Composition according to any of the claims 4 or 5, wherein the composition comprises ionic calcium in an amount of at least 1.7 mmol/L.

7. Composition according to any one of claims 1 to 6, wherein the oil mix comprises palmitic acid in an amount of at least 8% by weight based on total amount of total fatty acids.

8. Infant formula comprising the composition according to any of the claims 1 to 7, and wherein the ratio between the partially hydrolysed protein and the sn-2 palmitate is from 1.5:1 to 35:1 based on weight.

9. Infant formula according to claim 8, wherein the oil mix comprises palmitic acid in an amount of 8 to 30% by weight.

10. Infant formula according to claim 8, wherein the amount of partially hydrolysed proteins is at least 50% of the total amount of protein.

11. A composition according to any of the claims 1 to 7 or an infant formula according to any of the claims 8 to 10 for use in administration to an infant or young child to reduce calcium excretion by said infant or young child.

12. A composition according to any of the claims 1 to 7 or an infant formula according to any of the claims 8 to 10 for use in administration to an infant or young child to improve calcium homeostasis, increase calcium absorption, increase calcium retention, increase calcium utilization and/or reduce the formation of palmitic acid soaps.

13. A composition according to any of claims 1 to 7 or an infant formula according to any of the claims 8 to 10 for use in administration to an infant or young child to soften stools, prevent and/or reduce the risk of hard stools, prevent and/or reduce the risk of constipation, improve feeding tolerance, decrease the frequency and duration of crying and fussing, and alleviate digestive discomfort and colic.

14. A composition according to any of claims 1 to 7 or an infant formula according to any of the claims 8 to 10 for use in administration to an infant or young child to improve sleep duration, improve sleep quality and quantity, improve infant and parental quality of life and reduce maternal anxiety.

15. A composition according to any of claims 1 to 8 or an infant formula according to any of the claims 8 to 10 for use in administration to an infant or young child to increase bone mineralization, increase bone strength, and/or increase bone mineral density.

## Patentansprüche

1. Zusammensetzung zur Verwendung in Formulierungen für Säuglinge oder Kleinkinder, die teilweise hydrolysiertes Protein und eine Ölmischung umfasst, die zu Triacylglycerinen veresterte Palmitinsäure umfasst, wobei die in der sn-2-Position von Triacylglycerin veresterte Palmitinsäure in einer Menge von 20 Gew.-% bis 60 Gew.-%, bezogen auf das Gewicht der gesamten Palmitinsäure, und die in der sn-1(3)-Position von Triacylglycerin veresterte Palmitinsäure in einer Menge von 40 Gew.-% bis 80 Gew.-%, bezogen auf das Gewicht der gesamten Palmitinsäure, vorliegt; und wobei das Verhältnis zwischen dem teilweise hydrolysierten Protein und dem sn-2-Palmitat basierend auf dem Gewicht 1,5:1 bis 35:1 beträgt.

2. Zusammensetzung nach Anspruch 1, wobei es sich bei dem teilweise hydrolysierten Protein um teilweise hydrolysiertes Molkeprotein handelt.

3. Zusammensetzung nach Anspruch 1, wobei das teilweise hydrolysierte Protein einen Hydrolysegrad von 8 % bis 24 % aufweist.

4. Zusammensetzung nach einem der Ansprüche 1 bis 3, wobei die Zusammensetzung ferner ionisches Calcium umfasst.

5. Zusammensetzung nach Anspruch 4, wobei es sich bei dem ionischen Calcium um eines oder mehrere ausgewählt aus der Gruppe bestehend aus Calciumcitrat, Calciumhydroxid, Calciumoxid, Calciumchlorid, Calciumcarbonat, Calciumgluconat, Calciumphosphat, Calciumdiphosphat, Calciumtriphosphat, Calciumglycerophosphat, Calciumlactat und Calciumsulfat handelt.

6. Zusammensetzung nach einem der Ansprüche 4 oder 5, wobei die Zusammensetzung ionisches Calcium in einer Menge von mindestens 1,7 mmol/l umfasst.

7. Zusammensetzung nach einem der Ansprüche 1 bis 6, wobei die Ölmischung Palmitinsäure in einer Menge von mindestens 8 Gew.-%, bezogen auf die Gesamtmenge der gesamten Fettsäuren, umfasst.

8. Säuglingsformulierung, die die Zusammensetzung nach einem der Ansprüche 1 bis 7 umfasst und worin das Verhältnis zwischen dem teilweise hydrolysierten Protein und dem sn-2-Palmitat basierend auf dem Gewicht 1,5:1 bis 35:1 beträgt.

9. Säuglingsformulierung nach Anspruch 8, wobei die Ölmischung Palmitinsäure in einer Menge von 8 bis 30 Gew.-% umfasst.

10. Säuglingsformulierung nach Anspruch 8, wobei die Menge an teilweise hydrolysiertem Protein mindestens 50 % der Gesamtmenge an Protein beträgt.

11. Zusammensetzung nach einem der Ansprüche 1 bis 7 oder eine Säuglingsformulierung nach einem der Ansprüche 8 bis 10 zur Verwendung bei der Verabreichung an einen Säugling oder ein Kleinkind zum Verringern der Calciumausscheidung des Säuglings oder des Kleinkinds.

12. Zusammensetzung nach einem der Ansprüche 1 bis 7 oder eine Säuglingsformulierung nach einem der Ansprüche 8 bis 10 zur Verwendung bei der Verabreichung an einen Säugling oder ein Kleinkind zum Verbessern der Calciumhomöostase, zum Erhöhen der Calciumaufnahme, zum Erhöhen der Calciumretention, zum Erhöhen der Calciumverwendung und/oder zum Verringern der Bildung von Palmitinsäureseifen.

13. Zusammensetzung nach einem der Ansprüche 1 bis 7 oder eine Säuglingsformulierung nach einem der Ansprüche 8 bis 10 zur Verwendung bei der Verabreichung an einen Säugling oder ein Kleinkind zum Weichmachen des Stuhls, zum Verhindern und/oder Verringern der Gefahr von harten Stühlen, zum Verhindern und/oder Verringern der Gefahr von Verstopfung, zum Verbessern der Ernährungsverträglichkeit, zum Verringern der Häufigkeit und Dauer von Weinen und Jammern und zum Lindern von Verdauungsbeschwerden und Koliken.

14. Zusammensetzung nach einem der Ansprüche 1 bis 7 oder eine Säuglingsformulierung nach einem der Ansprüche 8 bis 10 zur Verwendung bei der Verabreichung an einen Säugling oder ein Kleinkind zum Verbessern der Schlafdauer, Verbessern der Schlafqualität und -quantität, zum Verbessern der Lebensqualität des Säuglings und der Eltern und zum Verringern von Mutterängsten.

15. Zusammensetzung nach einem der Ansprüche 1 bis 8 oder eine Säuglingsformulierung nach einem der Ansprüche 8 bis 10 zur Verwendung bei der Verabreichung an einen Säugling oder ein Kleinkind zur Steigerung der Knochenmineralisierung, zur Steigerung der Knochenfestigkeit und/oder zur Steigerung der Knochenmineraldichte.

## Revendications

1. Composition pour utilisation dans des préparations pour nourrissons ou jeunes enfants comprenant une protéine partiellement hydrolysée et un mélange d'huile comprenant de l'acide palmitique estérifié en triacylglycérols, dans laquelle l'acide palmitique estérifié à la position sn-2 du triacylglycérol est dans la quantité allant de 20 % à 60 % en poids d'acide palmitique total et l'acide palmitique estérifié à la position sn-1 (3) du triacylglycérol est dans la quantité allant de 40 % à 80 % en poids de l'acide palmitique total ; et dans laquelle le rapport entre la protéine partiellement hydrolysée et le sn-2 palmitate va de 1,5:1 à 35:1 sur la base du poids

2. Composition selon la revendication 1, dans laquelle la protéine partiellement hydrolysée est une protéine de lactosérum partiellement hydrolysée.

3. Composition selon la revendication 1, dans laquelle la protéine partiellement hydrolysée a un degré d'hydrolyse allant de 8 % à 24 %.

4. Composition selon l'une quelconque des revendications 1 à 3, dans laquelle la composition comprend en outre du calcium ionique.

5. Composition selon la revendication 4, dans laquelle le calcium ionique est un ou plusieurs choisis dans le groupe constitué de citrate de calcium, hydroxyde de calcium, oxyde de calcium, chlorure de calcium, carbonate de calcium, gluconate de calcium, phosphate de calcium, diphosphate de calcium, triphosphate de calcium, glycérophosphate de calcium, lactate de calcium et sulfate de calcium.

6. Composition selon l'une quelconque des revendications 4 ou 5, dans laquelle la composition comprend du calcium ionique en une quantité d'au moins 1,7 mmol/L.

7. Composition selon l'une quelconque des revendications 1 à 6, dans laquelle le mélange d'huile comprend de l'acide palmitique en une quantité d'au moins 8 % en poids sur la base de la quantité totale d'acides gras totaux.

8. Préparation pour nourrissons comprenant la composition selon l'une quelconque des revendications 1 à 7, et dans laquelle le rapport entre la protéine partiellement hydrolysée et le sn-2 palmitate va de 1,5:1 à 35:1 sur la base du poids.

9. Préparation pour nourrissons selon la revendication 8, dans laquelle le mélange d'huile comprend de l'acide palmitique en une quantité de 8 à 30 % en poids.

10. Préparation pour nourrissons selon la revendication 8, dans laquelle la quantité de protéines partiellement hydrolysées vaut au moins 50 % de la quantité totale de protéine.

11. Composition selon l'une quelconque des revendications 1 à 7 ou préparation pour nourrissons selon l'une quelconque des revendications 8 à 10 à utiliser dans l'administration à un nourrisson ou un jeune enfant pour réduire l'excrétion de calcium par ledit nourrisson ou jeune enfant.

12. Composition selon l'une quelconque des revendications 1 à 7 ou préparation pour nourrissons selon l'une quelconque des revendications 8 à 10 à utiliser dans l'administration à un nourrisson ou un jeune enfant de façon à améliorer l'homéostasie du calcium, augmenter l'absorption de calcium, augmenter la rétention de calcium, augmenter l'utilisation du calcium et/ou réduire la formation de savons d'acide palmitique.

13. Composition selon l'une quelconque des revendications 1 à 7 ou préparation pour nourrissons selon l'une quelconque des revendications 8 à 10 à utiliser dans l'administration à un nourrisson ou un jeune enfant pour ramollir les fèces, empêcher et/ou réduire le risque de fèces dures, empêcher et/ou réduire le risque de constipation, améliorer la tolérance à l'alimentation, diminuer la fréquence et la durée des pleurs et de l'agitation, et atténuer l'inconfort digestif et les coliques.

14. Composition selon l'une quelconque des revendications 1 à 7 ou préparation pour nourrissons selon l'une quelconque des revendications 8 à 10 à utiliser dans l'administration à un nourrisson ou un jeune enfant de façon à améliorer la durée de sommeil, améliorer la qualité et la quantité de sommeil, améliorer la qualité de vie du nourrisson et des parents et réduire l'anxiété maternelle.

15. Composition selon l'une quelconque des revendications 1 à 8 ou préparation pour nourrissons selon l'une quelconque des revendications 8 à 10 à utiliser dans l'administration à un nourrisson ou un jeune enfant pour augmenter la minéralisation osseuse, augmenter la solidité osseuse et/ou augmenter la densité osseuse en minéraux.
